# EUROPEAN PATENT SPECIFICATION

(11) **EP 0 299 434 B1**
(45) Date of publication and mention of the grant of the patent: **20.01.1993**
(21) Application number: 88111146.2
(22) Date of filing: 12.07.1988
(51) Int. Cl.: C07C 227/40, C07D 209/20

(54) **High pH recovery of neutral amino acids**
Isolierung von neutralen Aminosäuren bei hohen pH-Werten
Isolation d'amino acides neutres à des valeurs de pH élevées

(30) Priority: 17.07.1987 US 74832
(43) Date of publication of application: 18.01.1989
(73) Proprietor: GENENCOR INTERNATIONAL, INC., South San Francisco, CA 94080 (US)
(72) Inventor: Jaffari, Mark Darius c/o Eastman Kodak Company, Rochester New York 14650 (US)
(74) Representative: Brandes, Jürgen, Dr.

(56) References cited:
- EP-A- 0 140 713
- DE-B- 1 111 204
- US-A- 3 450 712
- PATENT ABSTRACTS OF JAPAN, unexamined applications, C field, vol. 8, no. 283, December 25, 1984 THE PATENT OFFICE JAPANESE GOVERNMENT page 71 C 258

## Description

The present invention is directed to a method for the recovery of a neutral amino acid from an aqueous solution. The method is capable of easily producing high purity acids without the need for organic solvents or expensive process steps.

In the description below, reference will be made to the neutral amino acid tryptophan. While tryptophan is a significant example of the invention, other neutral amino acids such as threonine and phenylalanine encounter similar problems in recovery, which problems are solved by the method of the invention. These acids share a characteristic solubility vs. pH curve (solubility increasing with increasing pH) as described more fully below.

Tryptophan is one of the essential amino acids. It is conveniently produced in commercial quantities by fermentation as well as by chemical methods. The tryptophan so produced is useful as an additive in human food products and animal feeds.

Whether produced by fermentation or chemical means, tryptophan is present in the final solution along with other materials. The most common method for removing these other materials is to adjust the pH of the fermentation broth to a low pH, remove the cell debris by centrifugation if a fermentation was used to produce the tryptophan and adsorb the tryptophan onto a strongly cationic ion exchange column. The tryptophan is eluted and then recovered from the eluting solution. In a common recovery, the solution is neutralized and then cooled at which time the desired product precipitates from the solution. While this method removes most of the impurities, many remain. It has thus been common practice to redissolve the precipitated solids in an organic solvent, for example ethanol, and then treat the solution with activated carbon. Reference is made to U.S. Patent 3,450,712 issued June 17, 1969 for a method of this type.

The use of an organic solvent is undesirable for several reasons. First, organic solvents are expensive. Also, since the tryptophan product is usually intended for animal or human consumption, organic solvents should be avoided since even trace amounts might be harmful.

As noted, in a typical method, a fermentation broth or other solution is first passed over an ion exchange column. The solution to be purified is first acidified to a pH of about 2 before being passed over the ion exchange column. The eluting solution is, for a cationic resin, a strong basic solution such as an ammonium hydroxide solution having a pH of about 11. The resultant elutant has a similar pH and is relatively dilute in tryptophan, e. g. about 10 g/L. Because of the solubility characteristics of tryptophan, recovery can be effected by acidifying the solution either by adding acid to the solution or by removing the base. The base can be removed where a volatile base such as ammonia is used. In the '712 patent, ammonia is removed when the dilute solution of tryptophan is concentrated under reduced pressure. It is the precipitate that is formed by this method that must be further purified by activated carbon from a methanol solution.

Another method for the recovery of tryptophan is disclosed in U.S. Patent 4,588,818. In this patent there is disclosed a method which produces what is referred to as the α-crystal form of tryptophan. A solution of tryptophan, for example a column eluate, is first filtered through a semipermeable membrane. An organic solvent is then added to this filtrate. Acid is then added to the solution to promote the formation of the desired crystals in such a manner that the crystals form from the alkaline side. Thus, this method also involves the use of expensive and undesirable organic solvents. Organic solvents are said to be necessary since they are said to relieve an inhibiting action to the formation of the desired α-type crystals.

In the working examples of the '818 patent it is clear that the method requires the organic solvent and that acid is added to effect neutralization. In both examples 1 and 2, hydrochloric acid is added to the solution in order to effect recovery.

Thus, the problem to be solved is to provide a method for the recovery of tryptophan that is inexpensive and that does not involve the use of organic solvents. The method should be capable of producing high purity tryptophan.

In accordance with the present invention there is provided a method for the recovery of a neutral amino acid from an aqueous solution characterized by the steps of:
1) forming a concentrated solution of said amino acid which solution has a pH above the solution pKa of the acid
2) removing base uniformly from the solution so as to form solid particles of the neutral amino acid at a pH above the solution pKa of the neutral amino acid.
3) recovering said solid particles from the solution.

In a preferred process, the aqueous solution is a fermentation broth. In an initial step, the broth is adjusted to a high pH and then concentrated and filtered at the high pH. Additional improvements are realized with this high pH filtration step. This step allows recycle of the supernatant and avoids expensive ion exchange.

Figure 1 is a plot of the saturation curve for tryptophan having illustrated thereon various purification schemes both of the prior art and of the invention.

Figure 2 is a plot of the course of the recovery of Example 3.

Figure 3 is a plot of the course of the recovery of Example 4.

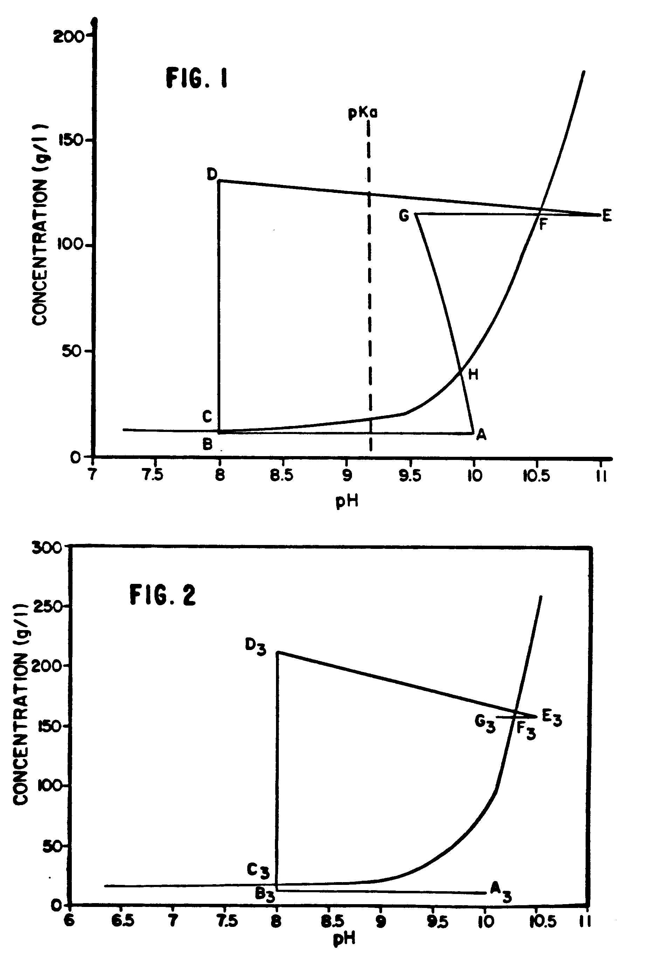

The present invention is best understood by reference to Fig. 1. Fig. 1 is a saturation curve for tryptophan. That is, points to the left of the curve (in Figures 1, 2 and 3) are conditions where the acid is not soluble and has precipitated out of the solution (or is in the form of a supersaturated solution) to form a slurry. Points to the right of the curve represent conditions where the acid is entirely in solution.

A typical eluate from a cation exchange resin is illustrated as point A. That is, the solution is relatively dilute in the acid and is at a high pH. The line A-B-C-D represents a typical prior art process where the ammonia eluate is concentrated by evaporation. In a process of this type, because of the volatility of ammonia, ammonia will come out of the solution easily and before there is significant concentration of the solution. As ammonia comes out of the solution, the pH decreases. Thus, the process moves from point A to B. If evaporation is continued, water will be removed and concentration of the solution will begin. Precipitation of the acid begins at C and the solids are recovered from the solution at D by filtration.

The solids at D are not as pure as desired. It is these solids that must be dissolved in organic solvent and then treated with activated carbon to obtain the desired product. It should be noted that the precipitation occurs at a pH that is well below the solution pKa of the acid which is at about 9.2 in this example.

In an alternative prior art method (not illustrated in Fig. 1), acid is added to a concentrated eluate solution and it may appear that solids begin to form while the bulk of the solution is above the solution pKa of the amino acid being recovered. However, the product formed in this manner is also of lesser purity than desired. It is theorized that as acid is added, the solids actually form in a localized low pH environment. In any event, adding acid to reduce the pH is not equivalent to uniformly removing base to reduce the pH as in the present invention.

Two embodiments of the present invention are illustrated in Fig. 1. In the first embodiment, the process starts out as does the prior art process. The elutant at A is taken to an amino acid slurry at D by evaporation. Rather than recovering the product at this point, the solids in the slurry are not removed but are redissolved by adding concentrated ammonium hydroxide or ammonia. The pH is raised, with some dilution if ammonium hydroxide solution is used, until point E is reached. Thus, a concentrated tryptophan solution having a pH above the solution pKa of tryptophan is formed. This corresponds to the first step of the invention summarized above.

From point E, ammonia is removed from the solution by evaporation or stripping until the solution reaches point G. That is, base is uniformly removed from the solution. Product solidification occurs at point F which is at a pH that is greater than the solution pKa of the amino acid at this temperature. Thus, step 2 of the invention is carried out. The solids at point G are harvested by filtration and need no additional purification. Unlike the solids at point D, they do not need to be redissolved in organic solvent and treated with activated charcoal.

A second embodiment of the invention is illustrated by the curve A-H-G in Figure 1. In this embodiment, the high pH ion exchange elutant at A is subjected to a reverse osmosis membrane. Unlike evaporation which removes the ammonia and the water sequentially, a reverse osmosis membrane will remove ammonia and water simultaneously. Thus, solids form at point H which again is above the solution pKa of the amino acid. In this embodiment, steps 1 and 2 of the invention are simultaneous.

It should be noted that under some circumstances, solids will not form precisely as the solution "crosses" the solubility curve. That is, a supersaturated solution might be formed. According to the invention, when solidification does begin to occur, the pH should, at that time, be above the solution pKa. Typically, a supersaturated solution will spontaneously solidify. Solidification can also be induced by introducing seed crystals into the supersaturated solution.

Thus, in accordance with preferred embodiments of the invention, the base is ammonium hydroxide and ammonia is uniformly removed in step 2) of the described method by volatilizing the ammonia. Thus, the base is uniformly removed by stripping it from the solution. The temperature during the solidification is not critical. Useful temperatures are between about 20°C and 60°C. Particularly preferred results are achieved when the temperature is about 55°C.

If desired, the solution can be subjected to vacuum to increase the rate of ammonia removal. Stirring of the solution and carrying the ammonia away with another gas, e.g. air, also promotes this process.

When base is stripped from the solution, a volatile base is needed. Ammonia (also in the form of ammonium hydroxide) is a convenient base for this purpose. Other volatile bases which can be used include hydrazine, ethylamine and pyridine.

In a second preferred method according to the invention, the base is also ammonium hydroxide and ammonia is uniformly removed while the tryptophan concentration of the solution is increased by reverse osmosis to simultaneously remove water and ammonia.

The reverse osmosis membrane should be selected so that ammonia is not removed at an appreciably greater rate than is the water. If ammonia is removed too rapidly, the pH will decrease to a point below the solution pKa of the amino acid before precipitation begins.

Examples of useful reverse osmosis membrane materials include: polyamide, polyacrylonitrile, polysulfone and polybenzimidazole. The shape of the semipermeable membrane is not critical and can be any of the conventional shapes known in the art. For example, the membrane can be tubular, flat sheets, and spiral or hollow fiber. Useful membranes have a salt rejection of greater or equal to 95% as measured by methods which are well known in the membrane art.

The method of the present invention provides for the inexpensive purification of neutral amino acids. In a typical application with tryptophan as the neutral amino acid, purities in excess of 95% can be attained without the use of additional steps requiring undesirable organic solvents.

The theoretical pKa of a neutral amino acid is a rigorously defined parameter which is independent of temperature and the presence of other components. In accordance with the present invention, the important pKa is the solution pKa which takes into account the actual characteristics of the solution. Thus, the pKa of the neutral amino acid in the actual solution from which it is desired to precipitate the amino acid must be measured. The "solution pKa" is the pH at which the solubility versus pH curve for the solution in question changes slope. For example, if the log of the concentration at saturation is plotted versus pH, two straight line portions of the curve will be noted. The intersection of these straight line portions when extended is considered to be the solution pKa of this solution under the conditions of the test. The solution pKa will depend on the other components in the solution, for example the base that might be present, the temperature and other factors.

The neutral amino acid can be recovered from any aqueous solution by the method of the invention. The solution can result from chemical methods for the amino acid production. As noted, the solution can be the eluate from an ion exchange column. In a preferred embodiment, the solution is a fermentation medium which has been concentrated and filtered at high pH so as to remove many impurities.

It has now been found that the cation exchange resin does not effectively remove impurities such as calcium ions and many proteins. A high pH concentration and filtration step removes most of these impurities while the desired neutral amino acid remains in solution. The amino acid is then recovered from this solution according to the invention.

One unexpected advantage of high pH concentration and filtration step is that the solution that results after precipitation can be recycled easily. If the starting solution is an ion exchange eluate, recycle is difficult due to the buildup of impurities that are not removed by the column. Thus, in a further preferred embodiment, the supernatant from the recovery is recycled.

To summarize the preferred embodiment of the invention, there is provided a method for the recovery of a neutral amino acid from a fermentation medium comprising the steps of:
1) concentrating and filtering the fermentation medium while maintaining a pH above about 10.0;
2) removing base uniformly from the solution so as to form solid particles of the neutral amino acid at a pH above the solution pKa of the neutral amino acid,
3) recovering said solid particles from the solution.

The solution resulting after solid particle recovery can be recycled to step 1.

The just described preferred method provides the desired amino acid at a purity approaching 100%. No organic solvents or ion exchange resins are needed and thus, the method is very inexpensive.

The following examples are presented for a further understanding of the invention.

In some of the following examples, an eluate from an ion exchange column is used. The ion exchange eluate was obtained as follows. Clarified fermentation broth (cells removed by filtration) was adjusted to a pH of about 2 with sulfuric acid. This solution was contacted with a strong cation exchange resin (Dowex 50 W X-2™) to adsorb the tryptophan that was in the fermentation broth. After washing the resin with water, the resin was eluted with 1 N ammonium hydroxide thereby removing the tryptophan from the resin. The resulting solution had a tryptophan concentration of about 12 g/L and a pH of about 10.

### Examples 1-3 Ammonia Stripping

The course of the Example is illustrated in Fig. 2. The starting point is the eluate at point A₃. The Example illustrated is Example 3 where the recovery was carried out at 55°C. The other recoveries at the other temperatures would be similar.

The ion exchange eluate was evaporated under vacuum at 50°-90°C to remove ammonia and water. The resulting solution had a tryptophan concentration of about 211 g/L and a pH of about 8.0. (point D₃) The tryptophan was partially precipitated so as to form a slurry.

To this slurry was added 14 M ammonium hydroxide so as to redissolve the tryprophan. The pH of the resulting solution was about 10.5. (point E₃) The solution was then heated and a stream of air was blown across the surface of the solution to carry away the ammonia while the solution was gently stirred. A supersaturated solution of tryptophan resulted (point G₃) which spontaneously solidified. The pH of the precipitation is shown in Table I. The solution pKa at each temperature is also given.

The slurry that resulted was filtered to remove the solid tryptophan. The solids were washed with cold water (0°-4°C). A portion of the solid was dried in a vacuum oven at 100°C and vacuum-dried for 16 hours. The purity of the resulting recovered tryptophan is indicated in Table 1.

**Table 1**

| Precipitation Conditions | | | |
|---|---|---|---|
| Ex. | Temperature | pH | Purity |
| 1 | 30°C (pKa= 9.0) | 9.9 | 97.6 |
| 2 | 39°C (pKa= 8.8) | 10.0 | 98.7 |
| 3 | 55°C (pKa= 8.4) | 10.1 | 99.8 |

### Example 4 Reverse Osmosis

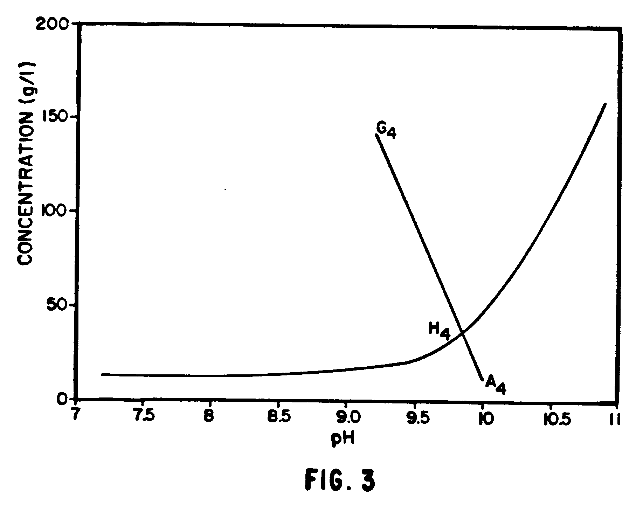

The course of this example is illustrated in Fig. 3.

Ten liters of the ion exchange eluate (Fig 3 point A₄) were concentrated with a reverse osmosis device. The device used was a DDS Lab Module 20 available from Niro Atomizer Food and Dairy Inc., Hudson, Wisconsin USA. The membrane area was 0.144 m² divided among four plates. Two of the plates were HR-95 membranes and two of the plates were HR-98 membranes both available from Niro.

The method was carried out at a pressure of about 5340 kPa, a recirculation rate of 1.8 L/min and a temperature of 37°C. The reverse osmosis was carried out until the concentrate stream reached a tryptophan concentration of 142 g/L at a pH of 9.2 at which point precipitation began (point G₄). The solution pKa of this solution is about 8.9.

The concentrated slurry was flushed out of the reverse osmosis device with about 500 mL of deionized water. The resulting slurry was allowed to cool to 23°C under mild stirring. It was then filtered under vacuum producing about 31 g of product. This product was washed with cold water. The product was dried as in Example 1. The dried sample was 97% pure tryptophan.

### Example 5 Acid Precipitation

This is a comparative example.

A portion of the ion exchange eluate was concentrated by evaporation to a tryptophan concentration of about 250 g/L. Sodium hydroxide was added during this step to keep the tryptophan in solution. The pH of this concentrate was about 10.8.

The concentrate was then added to a 50% by volume acetic acid solution at room temperature over a 30 minute time span. At the end point, the acetic acid:tryptophan ratio was 5:1. The solution was then stirred for an additional 30 minutes. During these steps, the tryptophan precipitated out of the solution and a slurry was formed.

The resulting slurry was filtered, washed with cold water and dried in a manner similar to example 1. The resulting solid had a tryptophan purity of only 90%.

In this example, the tryptophan was precipitated under acid conditions since the concentrate was added to a strongly acid solution. The pH of this solution was well below the pKa of the tryptophan concentrate. The purity of the resulting tryptophan was inferior to that produced by the method of the invention.

### Example 6 Evaporation Precipitation

This is a comparative example.

Two liters of the ion exchange eluate were evaporated at 60°C to a final volume of about 300 mL. The tryptophan concentration and pH of the resulting slurry were not measured but were estimated to be 69 g/L and 8.0 respectively. The tryptophan solution pKa under these conditions is about 8.2. Thus, precipitation ocurred well below the solution pKa.

The resulting slurry was filtered and the solids washed and dried as previously described. The purity of the tryptrophan that was produced was only 75.2%

### Example 7 High pH Filtration

A fermentation broth which had been filtered to remove the cell debris was used as the starting material in this example. No ion exchange purification had been performed. The sample of the filtered broth was 12.0 L and contained 5.4 g/L of tryptophan at a purity of 25% based on the solids present. The broth was concentrated using reverse osmosis to 2.5 L and 26.7 g/L tryptophan. The concentrated broth was then concentrated further by evaporation to a slurry of about 1.9 L.

The pH of the slurry was adjusted up to 10.3 using ammonia gas. The solids which remained at this pH were filtered off. Thus, the fermentation broth was filtered at high pH as is a preferred embodiment of the invention. The resulting filtrate was 1.75 L containing 36.4 g/L of tryptophan at a purity of 25.1%. The solids which were filtered off were 23.8 g of which 2.9% was tryptophan.

Two separate ammonia stripping precipitations were done on portions of the filtrate. The supernatants and washes from these two runs were recycled and used for another ammonia stripping precipitation. Since the filtrate from the high pH filtration was not at the desired concentration for ammonia stripping, it was concentrated by further evaporation. During this evaporation, more solids were formed which were filtered off at high pH.

From the three runs, a total of 14.6 g of tryptophan was recovered. Within experimental error, the purity of the product was 100% using both a high performance chromotography method and a titration method of determining purity.

## Claims

1. A method for the recovery of a neutral amino acid from an aqueous solution comprising the steps of:
1) forming a concentrated solution of said amino acid which solution has a pH above the solution pKa of the acid
2) removing base uniformly from the solution so as to form solid particles of the neutral amino acid at a pH above the solution pKa of the neutral amino acid.
3) recovering said solid particles from the solution.

2. A method according to claim 1 wherein said base is ammonium hydroxide and ammonia is uniformly removed in step 2) by volatilizing the ammonia.

3. A method according to claim 2 wherein said ammonia is volatilized using heat and vaccum.

4. A method according to claim 1 wherein said neutral amino acid is tryptophan.

5. A method according to claim 1 wherein the temperature during solidification is 55°C.

6. A method according to claim 1 wherein said base is ammonium hydroxide and ammonia is uniformly removed while the neutral amino acid concentration of the solution is increased by reverse osmosis to simultaneously remove water and ammonia.

7. A method according to claim 6 wherein said neutral amino acid is tryptophan.

8. A method for the recovery of a neutral amino acid from a fermentation medium comprising the steps of:
1) concentrating and filtering the fermentation medium while maintaining a pH above 10.0;
2) removing base uniformly from the solution so as to form solid particles of the neutral amino acid at a pH above the solution pKa of the neutral amino acid,
3) recovering said solid particles from the solution.

9. A method according to claim 8 wherein said neutral amino acid is tryptophan.

10. A method according to claim 8 wherein the solution resulting after solid particle recovery in step 3) is recycled to step 1.

## Patentansprüche

1. Verfahren zur Rückgewinnung einer neutralen Aminosäure aus einer wäßrigen Lösung mit den Stufen:
1) Bildung einer konzentrierten Lösung der Aminosäure mit einem pH-Wert oberhalb des Lösungs-pka der Säure
2) gleichförmige Entfernung von Base aus der Lösung unter Bildung fester Partikel der neutralen Aminosäure bei einem pH-Wert oberhalb des Lösungs-pKa der neutralen Aminosäure
3) Rückgewinnung der festen Partikel aus der Lösung.

2. Verfahren nach Anspruch 1, bei dem die Base Ammoniumhydroxid ist und Ammoniak gleichförmig in Stufe 2 durch Verflüchtigung des Ammoniaks entfernt wird.

3. Verfahren nach Anspruch 2, bei dem das Ammoniak durch Anwendung von Wärme und Vakuum verflüchtigt wird.

4. Verfahren nach Anspruch 1, bei dem die neutrale Aminosäure Tryptophan ist.

5. Verfahren nach Anspruch 1, bei dem die Temperatur während der Verfestigung bei 55°C liegt.

6. Verfahren nach Anspruch 1, bei dem die Base Ammoniumhydroxid ist und Ammoniak gleichförmig entfernt wird, während die neutrale Aminosäurekonzentration der Lösung erhöht wird durch Umkehrosmose unter gleichzeitiger Entfernung von Wasser und Ammoniak.

7. Verfahren nach Anspruch 6, bei dem die neutrale Aminosäure Tryptophan ist.

8. Verfahren zur Rückgewinnung einer neutralen Aminosäure aus einem Fermentationsmedium mit den Stufen:
1) Konzentrierung und Filtrieren des Fermentationsmediums unter Aufrechterhaltung eines pH-Wertes oberhalb 10,0;
2) gleichförmige Entfernung von Base aus der Lösung unter Bildung fester Partikel der neutralen Aminosäure bei einem pH-Wert oberhalb des Lösungs-pKa der neutralen Aminosäure,
3) Rückgewinnung der festen Partikel aus der Lösung.

9. Verfahren nach Anspruch 8, bei dem die neutrale Aminosäure Tryptophan ist.

10. Verfahre nach Anspruch 8, bei dem die Lösung, die nach der Rückgewinnung der festen Partikel in Stufe 3 hinterbleibt, zur Stufe 1 recyclisiert wird.

## Revendications

1. Procédé de récupération d'un aminoacide neutre dans une solution aqueuse, comprenant les étapes consistant à :
1) former une solution concentrée dudit aminoacide, laquelle solution a un pH supérieur au pKa en solution de l'acide
2) séparer uniformément la base de la solution de façon à former des particules solides d'aminoacide neutre à un pH supérieur au pKa en solution de l'aminoacide neutre
3) récupérer lesdites particules solides dans la solution.

2. Procédé selon la revendication 1, dans lequel ladite base est de l'hydroxyde d'ammonium et l'ammoniac est séparé uniformément à l'étape 2) par volatilisation.

3. Procédé selon la revendication 2, dans lequel ledit ammoniac est volatilisé par chaleur et vide.

4. Procédé selon la revendication 1, dans lequel ledit aminoacide neutre est le tryptophane.

5. Procédé selon la revendication 1, dans lequel la température durant la solidification est de 55°C.

6. Procédé selon la revendication 1, dans lequel ladite base est de l'hydroxyde d'ammonium et l'ammoniac est séparé uniformément cependant que la concentration en aminoacide neutre de la solution est augmentée par osmose inverse, pour que l'eau et l'ammoniac soient retirés simultanément.

7. Procédé selon la revendication 6, dans lequel ledit aminoacide neutre est du tryptophane.

8. Procédé de récupération d'un aminoacide neutre dans un milieu de fermentation, comprenant les étapes consistant à :
1) concentrer et filtrer le milieu de fermentation tout en maintenant un pH supérieur à 10,0 ;
2) séparer uniformément la base de la solution de façon à former des particules solides d'aminoacide neutre à un pH supérieur au pKa en solution de l'aminoacide neutre ;
3) récupérer lesdites particules solides dans la solution.

9. Procédé selon la revendication 8, dans lequel ledit aminoacide neutre est du tryptophane.

10. Procédé selon la revendication 8, dans lequel la solution obtenue après la récupération des particules solides à l'étape 3) est recyclée à l'étape 1.
